Europäisches Patentamt

European Patent Office

Office européen des brevets

(11) Veröffentlichungsnummer: **0 592 904 A1**

## (12) EUROPÄISCHE PATENTANMELDUNG

(21) Anmeldenummer: **93115941.2**

(22) Anmeldetag: **02.10.93**

(51) Int. Cl.5: **A61K 31/71**, A61K 47/12, A61K 47/20, A61K 47/28

(30) Priorität: **10.10.92 DE 4234225**

(43) Veröffentlichungstag der Anmeldung:
**20.04.94 Patentblatt 94/16**

(84) Benannte Vertragsstaaten:
**AT BE CH DE DK ES FR GB IT LI NL SE**

(71) Anmelder: **Röhm Pharma GmbH**
**Dr.-Otto-Röhm-Strasse 2-4**
**D-64331 Weiterstadt(DE)**

(72) Erfinder: **Wohlrab, Wolfgang A., Prof. Dr.**
**Pestalozzistrasse 35**
**D-06128 Halle(DE)**
Erfinder: **Neubert, Reinhard, Prof. Dr.**
**Schwuchtstrasse 11 f**
**D-06120 Halle(DE)**
Erfinder: **Matschiner, Sybille**
**Puschkinstrasse 18**
**D-06108 Halle(DE)**
Erfinder: **Wellner, Katrin, Dr.**
**Kleine Ulrichstrasse 3**
**D-06108 Halle(DE)**

(54) **Stabile topische Formulierungen mit gutem Wirkstoff-Freisetzungsvermögen enthaltend mindestens ein lipophilisiertes Makrolidantibiotikum.**

(57) Die Erfindung betrifft die Lipophilisierung von Makrolidantibiotika, insbesondere von Erythromycin und dessen Derivaten, mit Gegenionenbildnern, die einen oder mehrere lineare und/oder verzweigte Alkylsubstituenten mit 6 bis 32 Kohlenstoffatomen besitzen. Mit den Komplexen aus Makrolidantibiotikum und Gegenionenbildner werden stabile, bevorzugt halbfeste Formulierungen für topische Anwendungen mit einem guten Wirkstoff-Freisetzungsverhalten möglich. Bevorzugt weisen die Gegenionenbildner Alkylreste mit 12 bis 32 Kohlenstoffatomen und als ionenbildende Gruppen Sulfat-, Sulfonat- oder Salicylat-Gruppen auf.

EP 0 592 904 A1

Gebiet der Erfindung

Die Erfindung betrifft stabile topische Formulierungen mit gutem Wirkstoff-Freisetzungsvermögen, enthaltend mindestens ein Makrolidantibiotikum, das mit mindestens einem Gegenionenbildner, der einen oder mehrere lineare und/oder verzweigte Alkylsubstituenten aufweist, stabilisiert ist.

Stand der Technik

Makrolidantibiotika wie Erythromycin gehören zu der Gruppe von Pharmaka, die aufgrund ihrer hydrophilen Strukturelemente nur schlecht resorbiert werden (P. Langguth und E. Mutschler, Arzneim.-Forsch. 37 II (12), Seiten 1362 bis 1366, 1987).
H. Bojarska-Dahlig et al. (Curr. Chemother. Immunother. Proc. Int. Congr. Chemother. 12th, Meeting Date 1981, Vol.2, Seiten 898 bis 900, Ed. by P. Periti and G. Gialdroni-Grassi, Am. Soc. Microbiol., Washington D.C., 1982) beschreiben die Lipophilisierung von Erythromycin bzw. Erythromycin-Derivaten mit 1,3-Dioxolan-2-on-Einheiten und die dadurch bedingte Steigerung der antibakteriellen Wirkung von Erythromycin-Derivaten.
Der Einfluß der neueingeführten Substituenten auf Steifigkeit und Oberflächenbeschaffenheit des Erythromycin-Moleküls sowie die Auswirkungen dieser Parameter auf die Bildung ribosomaler Komplexe in Mirkoorganismen werden diskutiert.
EP-A 0 426 029 (US-Ser.No. 89/428 803) beschreibt ein Verfahren zur Reduktion der Lipophilie von Erythromycin bzw. von 6-O-Methyl-Erythromycin durch Inkorporieren o.g. Substanzen in Mizellen, die aus Steroiden wie Cholaten, Desoxycholaten, Glykocholaten und anderen aufgebaut sind. Es resultieren einspritzbare Emulsionen, mit denen Makrolidantibiotika intravenös verabreicht werden können. In Jpn. Kokai Tokkyo Koho JP 62 029 511 (= EP-A 211 250) werden lipophile Emulsionen von Erythromycin-Basen in einer wäßrigen Lösung von Phosphatid beschrieben, die unter Druck durch Zusatz von weiteren Emulgatoren hergestellt werden. Die lipophilen Teilchen in der wäßrigen Matrix weisen eine durchschnittliche Größe von ca. 0,1 $\mu$m auf.
WO 90/08 537 beschreibt Formulierungen zur oralen Verabreichung von pharmazeutischen Verbindungen, die einen therapeutisch wirksamen Anteil an Pharmazeutikum, ein organisches Lösungsmittel, ein Öl sowie gegebenenfalls lipophile Gegenionen, Stabilisatoren und Emulgatoren umfassen. Unter anderem enthalten die Formulierungen die schlecht resorbierbaren Antibiotika, wie beispielsweise Erythromycin, Doxorubicin, Gentamicin oder Tosufloxacin. Die orale Verfügbarkeit von Erythromycin-Base wird gegenüber den herkömmlichen Tablettenformulierungen auf das Doppelte angehoben .
EP 43 738 (= US 4 954 487) umfaßt Formulierungen zur topischen Verabreichung von pharmazeutischen Verbindungen, die ein Medium für den Transport des Pharmazeutikums durch intakte Haut enthalten, das aus (1) Diolen mit 3 bis 4 Kohlenstoffatomen, Diolestern oder Diolethern sowie aus (2) zellwandperturbierenden Substanzen, wie Alkenolen, Alkenylcarbonsäuren, Fettsäuren sowie Glyceride besteht. Eine Formulierung zur topischen Anwendung enthaltend Erythromycin erwies sich aufgrund der verbesserten Hautpenetration als effektives Pharmazeutikum zur Bekämpfung von Akne und weiteren systemischen Erkrankungen.

Aufgabe und Lösung

Derzeitig auf dem Markt befindliche Zubereitungen für topische Anwendungen von Makrolidantibiotika, insbesondere Erythromycin-Zubereitungen, weisen eine geringe Freisetzung und Stabilität der Wirksubstanz bzw. große Anteile an leicht flüchtigen Alkoholen auf, wie beispielsweise Ethanol oder 2-Propanol, wobei durch Flüchtigkeit eine Änderung der Wirksamkeit und Konsistenz der Zubereitung hervorgerufen wird.
Die im Stand der Technik angeführten Publikationen beziehen sich einerseits auf die Reduktion der Lipophilie von Makrolidantibiotika (EP-A 0 426 029), andererseits auf die Erhöhung der Liphophilie, wie sie für topische Anwendungen erwünscht ist.
Das von H. Bojarska-Dahlig et al. (siehe oben) beschriebene Verfahren zur Liphophilisierung von Erythromycin bzw. Erythromycin-Derivaten ist von der Synthese her aufwendig und zielt primär auf eine orale Anwendung mit hoher Blutaufnahmefähigkeit bei gleichzeitig ausreichender Magensaftresistenz des Antibiotikums.
Die von Jpn. Kokai Tokkyo JP 62 029511 umfaßten liphophilen Emulsionen werden parenteral verabreicht und sind für topische Anwendungen ebenso ungeeignet wie die in WO 90/08537 oral verabreichbaren Formulierungen in Gelatine-Kapseln, die neben dem Wirkstoff (Erythromycin) Öl, organisches Lösungsmittel sowie weitere Zusätze enthalten.
Die in EP 43 738 beschriebenen Formulierungen zur topischen Anwendung von Pharmazeutika enthalten beispielsweise Erythromycin als Wirkstoff, Diole oder Diol-Derivate sowie Alkenole und Alkenol-Derivate als Zusätze. Diese aufwendigen Formulierungen enthalten zu einem gewissen Anteil leichtflüchtige Substanzen, die zu o.g. Problemen bei der Langzeitstabilität führen.

Die daraus resultierende Aufgabe, lipophilisierte Makrolidantibiotika bzw. Formulierung enthaltend Makrolidantibiotika für topische Anwendungen mit gutem Wirkstoff-Freisetzungsverhalten zu finden, wird überraschenderweise von folgenden Makrolidantibiotika erfüllt.

Es wurde gefunden, daß Kombinationen aus Makrolidantibiotika und Gegenionenbildnern, ausgewählt aus der Gruppe der Alkylsulfate, Alkylsulfonate und Alkylsalicylate RX, wobei R für einen verzweigten oder linearen Alkylrest mit 6 bis 32 Kohlenstoffatomen und X für eine Sulfat-, Sulfonat- oder Salicylatgruppe steht, ausgezeichnete Stabilität und Freisetzungsverhalten in Salbenzubereitungen für topische Anwendungen aufweisen. Die Sättigungslöslichkeiten der erfindungsgemäßen Kombinationen aus Makrolidantibiotikum und Gegenion in dermatologisch interessanten Vehikeln, wie beispielsweise Propylenglykol, sind gegenüber dem unmodifizierten Makrolidantibiotikum deutlich erhöht.

Die Makrolidantibiotika

Makrolidantibiotika besitzen eine ausgezeichnete antibakterielle Wirkung speziell gegen grampositive Bakterien. Die Klassifizierung der Makrolidantibiotika erfolgt nach der Größe der makrozyklischen Lactonringe. Makrolidantibiotika sind polyfunktionelle Moleküle, wobei die Mehrzahl mindestens eine Aminozuckereinheit aufweist und basisch ist (vgl. Kirk-Othmer, Encyclopedia of Chemical Technology, 3rd. Ed., Vol. 2, Seite 937, J. Wiley, 1978).

Sie werden als sekundäre Metaboliten von Mikroorganismen, die bevorzugt im Erdreich zu finden sind, erzeugt, besonders bevorzugt von den verschiedenen Streptomyces-Stämmen. Makrolidantibiotika werden technisch durch aerobe Submers-Fermentation geeigneter Kulturen bei 20 bis 40 Grad C als komplexes Gemisch verwandter Verbindungen gewonnen. Die Isolierung erfolgt nach Einstellung des pH der Fermentationslösung auf 9,5 durch Extraktion mit geeigneten Lösungsmitteln wie beispielsweise Ethylacetat, Chloroform oder Methylenchlorid sowie durch anschließende Ausfällung des kristallinen Rohprodukts, wobei eine chromatographische Feinaufarbeitung nachfolgt (Kirk-Othmer loc.cit., Seite 940).

Beispielhaft für Makrolidantibiotika mit 12-gliedrigen Lactonringen seien Methylmycin (der erste Makrolid, dessen Struktur bestimmt wurde) oder Neomethylmycin genannt. Unter den Makrolidantibiotika mit 14-gliedrigen Lactonringen seien bevorzugt die Erythromycine genannt, die von den Streptomyces erythreus erzeugt werden. Beispielhaft seien angeführt: Erythromycin A, Erythromycin B, Erythromycin C, Erythromycin D, Erythromycin E, Erythromycin-Estolat, Erythronolid B oder Clarithromycin. Weitere Beispiele für Makrolidantibiotika mit 14-gliedrigen Lactonringen sind: Megalomycin und Megalomycin-Derivate, Picromycin, Narbomycin, Oleandomycin, Triacetyloleandomycin, sowie die neutralen Verbindungen Laukamycin, Kujimycin A, Albocyclin und Cineromycin B.

Als Vertreter für Makrolidantibiotika mit 16-gliedrigen Lactonringen seien beispielhaft genannt: Magmamycin (= Carbomycin) und Derivate, wie beispielsweise Niddamycin, Spiramycin und dessen Derivate, Leucomycin und Leucomycin-Derivate, wie beispielsweise Midecamycin, Maridomycin, Tylosin, Cirramycin, Juvenimycine, sowie als neutrale Vertreter Chalcomycin und Neutramycin (loc.cit., Seiten 941 bis 950).

Beispiele für Makrolidantibiotika mit größeren Lactonringen, beispielsweise mit 26 bis über 40 Ringgliedern, sind: Pimaricin, Lucensomycin, Nystatin, Amphotericin B, Hamycin, Candicidin A und B, Candidin sowie Levorin. Die Wirkung dieser Gruppe richtet sich praktisch ausschließlich gegen Pilze und Hefen. Bakterien werden nicht oder kaum beeinflußt, wobei die Toxizität o.g. Substanzen durchweg niedrig ist (vgl. Ullmanns Enzyklopädie der technischen Chemie, 4. Aufl., Bd. 9, Seite 673, 674, Verlag Chemie, Weinheim, 1980).

Im klinischen Bereich werden Makrolidantibiotika in der Hauptsache zur Bekämpfung von Infektionen mit Streptokokken, Staphylokokken und Pneumokokken eingesetzt. Die Toxizität der Makrolidantibiotika ist im allgemeinen niedrig. Therapeutische Bedeutung erlangt haben Ester von Makrolidantibiotika, da sie nach oraler Gabe rasch höhere Blutspiegel erzeugen, praktisch geruchs- und geschmacksfrei sind und eine hohe Stabilität besitzen.

Die lipophilisierenden Gegenionenbildner

In WO 90/08537 werden "lipophile Gegenionen" beschrieben, die durch organische Säuren oder deren Salze gebildet werden, deren pka-Wert ausreichend niedrig ist, um eine Dissoziation bei den in den Formulierungen herrschenden pH-Werten zu gewährleisten. Die organischen Säuren umfassen Ölsäure, Alkylsulfonsäuren, Salicylsäure, Gallensäuren (Säuren von bestimmten Steroiden), Palmitinsäure und andere aliphatische Säuren.

Die erfindungsgemäßen Gegenionenbildner umfassen Alkylsulfate, Alkylsulfonate und Alkylsalicylate RX, wobei R für einen linearen und/oder verzweigten Alkylsubstituenten mit 6 bis 32 Kohlenstoffatomen und X für eine Sulfat-, Sulfonat- oder Salicylat-Gruppe steht. Bevorzugt sind Alkylsubstituenten R mit 12 bis 32 Kohlenstoffatomen, besonders bevorzugt Alkylsubstituenten mit 16 bis 24 Kohlenstoffatomen. Bevorzugte Gruppen X sind die Sulfat- und die Sulfonat-Gruppe.

Beispielhaft für die Gegenionenbildner seien genant: Hexylsalicylat, Heptylsalicylat, Octylsalicylat, Isooctylsalicylat, Nonylsalicylat, Decylsalicylat, Isodecylsalicylat, Undecylsalicylat, Dodecylsalicylat, Tetradecylsalicylat, Isotetradecylsalicylat, Hexadecylsalicylat, Octadecylsalicylat, Eicosylsalicylat, Hexylsulfat, Octylsulfat, Isooctylsulfat, Decylsulfat, Isodecylsulfat, Dodecylsulfat, Tetradecylsulfat, Isotetradecylsulfat, Hexadecylsulfat, Octadecylsulfat, Eicosylsulfat, Hexylsulfonat, Octylsulfonat, Isooctylsulfonat, Decylsulfonat, Isodecylsulfonat, Dodecylsulfonat, Tetradecylsulfonat, Isotetradecylsulfonat, Hexadecylsulfonat, Octadecylsulfonat, Eicosylsulfonat.

Die besondere Wirkung der Gegenionenbildner auf die Lipophilisierung von Makrolidantibiotika kann durch das Verteilungsgleichgewicht im System n-Octanol/wäßriger Phosphatpuffer, charakterisiert durch den Verteilungskoeffizienten P = Quotient aus Konzentration des Makrolidantibiotikums in n-Octanol (Zähler) und Konzentration des Makrolidantibiotikums im wäßrigen Phosphatpuffer (Nenner), veranschaulicht werden. Der Betrag von P ist ein Maß für die Lipophilie der Kombination aus Makrolidantibiotikum und Gegenionenbildner. Untersucht wurde das Verteilungsgleichgewicht von Erythromycin-Base im System n-Octanol/wäßriger Phosphatpuffer für verschiedene Gegenionen, wobei ein physiologisch relevanter pH-Bereich zwischen pH = 6,0 (resorbierende Hautoberfläche) und pH = 7,4 (mittlerer pH-Wert des Blutes) überstrichen wurde. Tabelle 1 zeigt, daß von den untersuchten Gegenionenbildnern insbesondere Dodecylsulfat und Octadecylsulfonat geeignet sind, ein vom pH-Wert weitgehend unabhängiges Verteilungsgleichgewicht mit einem sehr hohen Anteil an Erythromycin + Gegenion in der lipophilen Phase einzustellen. Ein Vergleich mit Gegenionenbildnern des Standes der Technik wie Desoxycholat, Dehydrocholat, Octylcarbonsäure, Dodecylcarbonsäure oder Cyclamat zeigt die Überlegenheit der erfindungsgemäßen Gegenionenbildner bei der Lipophilisierung von Erythromycin-Base. Die Verteilungskoeffizienten P sind deutlich höher und vom pH-Wert der wäßrigen Phase weitestgehend unabhängig.

Die Untersuchung der Dicarbonsäure Azelainsäure erfolgte aufgrund ihrer positiven Wirkung als Akne-Therapeutikum. Als liphophilisierender Gegenionenbildner ist sie, insbesondere bei niedrigen pH-Werten ungeeignet.

Eine Betrachtung der Verteilungskoeffizienten P von Erythromycin in Abhängigkeit der Konzentration der Gegenionenbildner bei konstantem pH = 7,4 zeigt, daß neben dem erfindungsgemäß vorteilhaft wirkenden Dodecylsulfat und Octadecylsulfonat auch das Hexylsalicylat, welches bei der pH-abhängigen Betrachtung eine nur wenig überlegene Wirkung zeigt, ab einem Verhältnis von Erythromycin : Gegenionenbildner von 1 : 2 sehr gut lipophilisierend wirkt (Tabelle 2).

Freisetzungsuntersuchungen an halbfesten Zubereitungen enthaltend lipophilisiertes Erythromycin

Halbfeste Zubereitungen, wie beispielsweise Cremes, Salben oder Pasten, sind bevorzugt Formulierungen topisch wirkender Makrolidantibiotika.

Grundlage für Cremes sind beispielsweise wachsartige Substanzen, die aufgeschmolzen werden, mit dem Wirkstoff versetzt werden und unter Abkühlen gerührt werden. Im allgemeinen wird die Formulierung danach in einer Rollmühle zur Einstellung einer bestimmten Teilchengröße des dispergierten Wirkstoffs fortgeführt. Pasten werden gleichermaßen hergestellt, sie weisen nur einen höheren Feststoffgehalt auf.

Cremes sind halbfeste Wasser-in-Öl- bzw. Öl-in-Wasser-Emulsionen. Im allgemeinen werden die Bestandteile beider Phasen separat auf 70 bis 80 Grad C aufgeheizt und danach unter Abkühlen zur Emulsion verrührt. Um die emulgierten Tröpfchen weiter zu verkleinern, wird die Emulsion vor dem endgültigen Abkühlen mit einer Homogenisierapparatur, beispielsweise einer Kolloidmühle, aufbereitet (vgl. z.B. Kirk-Othmer, Encyclopedia of Chemical Technology, 3rd. Vol. 17, Seite 285, J. Wiley, 1982).

Als halbfeste Zubereitungen der erfindungsgemäßen liphophilisierten Makrolidantibiotika werden vorzugsweise stabile Salbenzubereitungen angestrebt. Dazu wurden zunächst die für topische Anwendungen wichtigen Sättigungslöslichkeiten in dermatologisch interessanten Vehikeln untersucht. Hierbei ist die Zersetzung von Erythromycin im wäßrigen Milieu zu berücksichtigen, was einen Ausschluß von Wasser als Vehikelbestandteil notwendig macht, sowie beim Einsatz als Akne-Therapeutikum die Verwendung von weitestgehend fettfreien Vehikeln.

Aus einer Vielzahl von untersuchten Lösungsmitteln, wie beispielsweise Paraffinen, Miglyol, Octansäure, Isopropylmyristat, Palmitinsäureisopropylester, Ethylenglykol, Hexadecan, Polyethylenglykol 400 sowie anderen, wurden im Hinblick auf stabile Salbenzubereitungen Propylenglykol, Glycerin und Hexylenglykol als besonders wirksam aufgefunden. Tabelle 3 zeigt die Sättigungslöslichkeiten von Erythromycin bzw. von den Erythromycin-Gegenion-Komplexen Erythromycinoctylsulfonat und Erythromycinhexylsalicylat in den Lösungsmitteln Glycerin und Propylenglykol, die als Beispiele für dermatologisch interessante Vehikel anzusehen sind. Während in Glycerin nur Erythromycinoctylsulfonat eine gegenüber Erythromycin verbesserte Sättigungslöslichkeit zeigt, weisen beide betrachteten Erythromycin-Gegenion-Komplexe in Propylenglykol eine gegenüber Erythromycin in Propylenglykol sowie gegenüber Erythromycin und Erythromycin-Komplexen in Glycerin deutlich verbesserte Sättigungslöslichkeit auf. Erythromycinoctadecylsulfonat weist im Hexylenglykol eine ausgezeichnete Sättigungslöslichkeit auf.

Die Freisetzungsuntersuchungen erfolgen am Mehrschichtmembranmodell (R. Neubert und W. Wohlrab, Acta Pharm. Technol. 36 (4), Seiten 197 bis 200, 1990), wobei die Analytik nach dem Verfahren nach Dabrowska et al. (Sci. Pharm. 52, Seiten 220 bis 228, 1984) durchgeführt wurde. Gearbeitet wurde mit 4 Membranen, wobei die erste Membran aus getrocknetem mit Dodecanol und Phosphatpuffer (pH-Wert = 5,5) gesättigtem Nephrophan® besteht sowie die drei weiteren sich anschließenden Membranen aus einem Gemisch aus 50 Gew.-% Collodium und 50 Gew.-% Dodecanol bestehen. Nach dem Auftragen der Salben (topische Zubereitungen mit jeweils 2 % Erythromycin) und der Temperierung bei 32 Grad C und während einer Dauer von 200 Minuten wurde jede Membran mit 0,2 ml einer 0,1 N NaOH-Lösung und 3 ml Chloroform versetzt und 30 Minuten geschüttelt. Die Chloroformphase wurde abgetrennt und mit einer Mischung aus 3 ml Phosphatpuffer (pH = 5,3) und 3 ml wäßriger Bromkresolpurpurlösung (zu 0,1727 g Bromkresolpurpur werden 4 ml 0,1 M Natriumhydroxid-Lösung gegeben und mit destillierte Wasser auf 200 ml aufgefüllt und danach filtriert) nochmals 30 Minuten geschüttelt. Danach wurde die Chloroformphase mit 2 ml 0,1 N NaOH geschüttelt und die wäßrige Phase bei 590 nm photometrisch vermessen. Die Wiederfindungsrate von Erythromycin in den Dodecanol-Collodium-Membranen lag bei 96,2 %.

In Tabelle 4 sind die Erythromycin-Gehalte in den Membranen summarisch zu verschiedenen Zeiten angegeben. Untersucht wurden jeweils Formulierungen mit 2 % Erythromycin-Wirkstoffgehalt: die erfindungsgemäße Formulierung mit dem Komplex Erythromycinoctadecylsulfonat, Zineryt ®-Salbe der Fa. Röhm Pharma als Referenz. Zineryt ® dient zur Akne-Therapie und enthält 2 % Erythromycin als Wirkstoff. Die erfindungsgemäßen Formulierungen weisen bessere topische Verfügbarkeiten entsprechend den Anreicherungen in den 4 Membranen auf. Als im Freisetzungsverhalten von Erythromycinoctadecylsulfonat besonders wirksame Salbenformulierung ist die Formulierung III in Tabelle 5 anzusehen, die als Salbengrundlage Propylenglykol, Hexylenglykol, Palmitinsäure und Glycerin enthält.

Vorteile der Erfindung

Die mit den erfindungsgemäßen Gegenionenbildnern lipophilisierten Makrolidantibiotika weisen ausgezeichnete Löslichkeiten in lipophilen Medien auf, wobei die Löslichkeit bei Kontakt mit wäßrigen Medien über den physiologisch interessanten pH-Bereich zwischen 6,0 und 7,4 konstant hoch bleibt. Bei Gegenionenkomplexen des Standes der Technik ist dagegen ein starker Rückgang des lipophilen Charakters der Makrolidantibiotika bei Kontakt mit wäßrigen Medien festzustellen.

Aus halbfesten Zubereitungen, wie sie bevorzugt für topische Anwendungen eingesetzt werden, zeigen die erfindungsgemäßen Makrolidantibiotikum-Gegenionenbildner-Komplexe ein gegenüber marktüblichen Formulierungen überlegenes Freisetzungsverhalten.

Die Stabilität der erfindungsgemäßen Komplexe in wäßrigen Zubereitungen ist gegenüber den reinen Makrolidantibiotika, insbesondere ist dies für Erythromycin der Fall, deutlich höher.

TABELLE 1

Verteilungskoeffizient P von Erythromycin in Abhängigkeit von der Art des Gegenionenbildners und dem pH-Wert des wäßrigen Phosphatpuffers im System n-Octanol/wäßriger Phosphatpuffer

| Gegenionenbildner (molares Verhältnis Gegenionenbildner/ Erythromycin = 1 : 1) | $P$ | | |
|---|---|---|---|
| | pH = 6,0 | 6,8 | 7,4 |
| Octadecylsulfonat | $28,5 \pm 2,1$ | $29,1 \pm 3,3$ | $27,1 \pm 2,5$ |
| Dodecylsulfat | $20,6 \pm 1,7$ | $21,7 \pm 1,1$ | $20,1 \pm 0,7$ |
| Hexylsalicylat | $6,2 \pm 0,7$ | $10,5 \pm 0,3$ | $10,5 \pm 0,4$ |
| Vergleichsbeispiele: | | | |
| Desoxycholat | $5,4 \pm 0,7$ | $9,2 \pm 0,3$ | $10,1 \pm 0,5$ |
| Dehydrocholat | $0,3 \pm 0,1$ | $4,5 \pm 0,2$ | $10,9 \pm 0,2$ |
| Octylcarbonsäure | $4,9 \pm 0,1$ | $7,5 \pm 1,1$ | $15,1 \pm 0,2$ |
| Dodecylcarbonsäure | $< 0,01$ | $4,2 \pm 0,8$ | $5,9 \pm 0,2$ |
| Cyclamat | $4,2 \pm 0,7$ | $8,7 \pm 0,6$ | $9,7 \pm 0,2$ |
| Azelainsäure | $< 0,01$ | $4,2 \pm 0,8$ | $5,3 \pm 0,3$ |
| Ohne Gegenionenbildner | $0,2 \pm 0,2$ | $2,9 \pm 1,2$ | $5,5 \pm 0,4$ |

*TABELLE 2*

*Verteilungskoeffizient P von Erythromycin in Abhängigkeit der Konzentration des Gegenionenbildners im System n-Octanol/wäßriger Phosphatpuffer bei pH = 7,4*

| | $P$ | | |
|---|---|---|---|
| *Gegenionenbildner* | *1 : 1* | *1 : 2* | *1 : 5* |
| | *(molares Verhältnis Erythromycin : Gegenionenbildner)* | | |
| *Octadecylsulfonat* | *27,1 ± 2,5* | *30,1 ± 3,6* | *28,3 ± 2,5* |
| *Dodecylsulfat* | *20,1 ± 0,7* | *23,8 ± 1,3* | *22,3 ± 1,4* |
| *Hexylsalicylat* | *10,5 ± 0,4* | *24,4 ± 3,2* | *21,0 ± 1,0* |
| *Vergleichsbeispiele:* | | | |
| *Desoxycholat* | *10,1 ± 0,5* | *9,6 ± 1,2* | *11,2 ± 0,9* |
| *Dehydrocholat* | *10,9 ± 0,2* | *14,8 ± 2,2* | *17,5 ± 1,6* |
| *Octylcarbonsäure* | *15,1 ± 2,0* | *12,9 ± 1,3* | *14,1 ± 0,6* |
| *Dodecylcarbonsäure* | *5,9 ± 0,2* | *5,7 ± 0,1* | *6,3 ± 0,5* |
| *Cyclamat* | *9,7 ± 0,2* | *9,6 ± 0,8* | *9,5 ± 1,4* |
| *Azelainsäure* | *5,3 ± 0,2* | *5,4 ± 0,2* | *5,9 ± 0,3* |
| *ohne Gegenionenbildner* | *5,5 ± 0,4* | | |

TABELLE 3

| Sättigungslöslichkeiten SL von Erythromycin (E) bzw. den liphophilisierten Komplexen Erythromycinhexylsalicylat ($E^+HS^-$), Erythromycinoctylsulfonat ($E^+OS^-$) und Erythromycinoctadecylsulfonat ($E^+ODS^-$) in dermatologisch interessanten Vehikeln | | | | |
|---|---|---|---|---|
| Vehikel | SL in mg/ml | | | |
| | E | $E^+HS^-$ | $E^+OS^-$ | $E^+ODS^-$ |
| Glycerin | 1,9 | 1,6 | 3,4 | - |
| Propylenglykol | 6,7 | 12,2 | 20,8 | - |
| Hexylenglykol | - | - | - | > 20 |
| (SL wurde nach bekannter Methode (z.B. Römpps Chemielexikon, 9. Aufl., Bd. 3, Seiten 2537 bis 2540, Thieme-Verlag, Stuttgart, 1990) bestimmt, wobei die Proben 3 Stunden bei 32 Grad C temperiert und im Abstand von 10 min geschüttelt wurden). | | | | |

TABELLE 4

Versuche zum Freisetzungsverhalten von Erythromycin-Wirkstoff am Mehrschichtmembranmodell: Vergleich mit einem marktgängigen Produkt.

1. Membran:                    Nephrophan ® mit Dodecanol gesättigt, getrocknet und
                               mit wäßrigem Phosphatpuffer pH 5,5 gesättigt

2 bis 4. Membran:              50 Gew.-% Collodium, 50 Gew.-% Dodecanol

| Zeit/Minuten | Formulierung | Gehalt an Erythromycin bzw. Komplex Erythromycin-Gegenion bezogen auf die ursprünglich eingesetzte Menge Erythromycin (in %) | |
|---|---|---|---|
| | | Summe: Membran 1 - 4 | Membran 2-4 |
| 15 | I | 19,9 | 8,6 |
| | II | 20,3 | 9,0 |
| | ZE | 7,3 | 1,4 |
| 30 | I | 33,2 | 17,3 |
| | II | 28,8 | 15,0 |
| | ZE | 10,4 | 1,9 |
| 60 | I | 53,4 | 32,4 |
| | II | 49,1 | 30,6 |
| | ZE | 20,8 | 9,8 |

Legende:

I:  Salbengrundlage:        15 Teile Macrogolstearat
                            10 Teile Propylenglykol
                            75 Teile Glycerin

    2,8 % Erythromycinoctadecylsulfonat entsprechend 2 % Erythromycin

II: Salbengrundlage:        15 Teile Macrogolstearat
                            10 Teile Propylenglykol
                            72 Teile Glycerin
                             3 Teile Zinkoxid

    2,8 % Erythromycinoctadecylsulfonat entsprechend 2 % Erythromycin

ZE: Zineryt ® der Fa. Röhm Pharma enthaltend 2 % Erythromycin

8

*TABELLE 5*

*Versuche zum Freisetzungsverhalten von Erythromycin-Wirkstoff am Mehrschicht-membranmodell zum Auffinden einer besonders gut wirksamen Salbenrezeptur.*

*1. Membran:*       *84 Gew.-% Collodium, 16 Gew.-% Dodecanol getrocknet*
*mit wäßrigem Phosphatpuffer pH 5,5 gesättigt*

*2. bis 4. Membran:*      *50 Gew.-% Collodium, 50 Gew.-% Dodecanol*

*Zeit/Minuten*      *Formulierung*      *Gehalt an Erythromycinoctadecylsulfonat bezogen auf die ursprünglich eingesetzte Menge in %*

| Zeit/Minuten | Formulierung | Summe: Membran 1 - 4 | Membran 2 - 4 |
|---|---|---|---|
| *15* | *I* | *25,0* | *8,8* |
| | *II* | *21,6* | *7,9* |
| | *III* | *34,6* | *10,8* |
| | *IV* | *21,8* | *7,3* |
| *30* | *I* | *35,6* | *15,6* |
| | *II* | *41,7* | *17,8* |
| | *III* | *57,8* | *18,4* |
| | *IV* | *36,7* | *11,0* |
| *60* | *I* | *52,0* | *16,4* |
| | *II* | *60,5* | *20,6* |
| | *III* | *69,9* | *23,1* |
| | *IV* | *62,4* | *26,6* |

*Legende:*

*I:*   *Salbengrundlage*      *15 Teile Macrogolstearat*
     *(entsprechend II*      *10 Teile Propylenglykol*
     *in Tabelle 4)*      *72 Teile Glycerin*
     *3 Teile Zinkoxid*

*II: Salbengrundlage:*      *15 Teile Macrogolstearat*
     *10 Teile Propylenglykol*
     *75 Teile Glycerin*

*III:Salbengrundlage*      *10 Teile Propylenglykol*
     *10 Teile Hexylenglykol*
     *5 Teile Palmitinsäure*
     *75 Teile Glycerin*

*IV: Salbengrundlage*      *15 Teile Cetylstearylalkohol*
     *10 Teile Propylenglykol*
     *10 Teile Hexylenglykol*
     *65 Teile Glycerin*

*Alle Salbenformulierungen II bis IV enthalten als Wirkstoff 2,8 % Erythromycin-octadecylsulfonat entsprechend 2 % Erythromycin bezogen auf die Gesamtformulierung.*

## Patentansprüche

1. Stabile topische Formulierungen mit gutem Wirkstoff-Freisetzungsverhalten enthaltend mindestens ein Makrolidantibiotikum, das mit mindestens einem Gegenionbildner lipophilisiert ist,
   dadurch gekennzeichnet,
   daß der Gegenionenbildner ausgewählt ist aus der Gruppe Alkylsulfat, Alkylsulfonat und Alkylsalicylat RX, wobei R für einen linearen und/oder verzweigten Alkylrest mit 6 bis 32 Kohlenstoffatomen und X für eine Sulfat-, Sulfonat- oder Salicylat-Gruppe steht.

2. Formulierungen gemäß Anspruch 1, dadurch gekennzeichnet, daß der lineare und/oder verzweigte Alkylsubsitutent des Gegenionenbildners 12 bis 32 Kohlenstoffatome aufweist.

3. Formulierungen gemäß den Ansprüchen 1 bis 2, dadurch gekennzeichnet, daß das Makrolidantibiotikum Erythromycin und/oder ein Erythromycin-Derivat ist.

4. Verwendung der Formulierungen gemäß den Ansprüchen 1 bis 3 in halbfesten Zubereitungen.

5. Halbfeste Zubereitungen gemäß Anspruch 4, dadurch gekennzeichnet, daß die halbfesten Zubereitungen Salbenzubereitungen sind, enthaltend eine oder mehrere Substanzen aus der Gruppe Propylenglykol, Glycerin, Hexylenglykol, Cetylstearylalkohol, Macrogolstearat und Palmitinsäure.

**Europäisches Patentamt**

**EUROPÄISCHER RECHERCHENBERICHT**

Nummer der Anmeldung

**EP 93 11 5941**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,Y | WO-A-90 08537 (ABBOTT)<br>* Ansprüche 1,5-14 *<br>* Seite 4, Zeile 6 - Zeile 14 *<br>* Seite 4, Zeile 25 - Zeile 33 *<br>* Seite 5, Zeile 1 - Zeile 5 *<br>* Beispiel 4 *<br>--- | 1-5 | A61K31/71<br>A61K47/12<br>A61K47/20<br>A61K47/28 |
| D,Y | EP-A-0 043 738 (PROCTER & GAMBLE)<br>* Ansprüche 1,6-8 *<br>* Seite 6, Zeile 10 - Zeile 35 *<br>* Seite 7, Zeile 1 - Zeile 12 *<br>* Seite 7, Zeile 24 - Zeile 28 *<br>* Seite 13, Zeile 13 - Zeile 15 *<br>* Beispiel XVII *<br>--- | 1-5 | |
| Y | DE-A-19 44 906 (ABBOTT)<br>* Ansprüche *<br>* Beispiele *<br>--- | 1-5 | |
| D,A | EP-A-0 426 029 (ABBOTT)<br>* das ganze Dokument *<br>--- | 1-5 | **RECHERCHIERTE SACHGEBIETE (Int.Cl.5)** |
| D,A | EP-A-0 211 258 (ABBOTT)<br>* das ganze Dokument *<br>----- | 1-5 | A61K |

Der vorliegende Recherchenbericht wurde für alle Patentansprüche erstellt

| Recherchenort | Abschlußdatum der Recherche | Prüfer |
|---|---|---|
| DEN HAAG | 13. Januar 1994 | SCARPONI, U |

EPO FORM 1503 03.82 (P04C03)